(19) **Europäisches Patentamt / European Patent Office / Office européen des brevets**

(11) **EP 4 570 174 A1**

(12) **EUROPEAN PATENT APPLICATION**

(43) Date of publication:
**18.06.2025  Bulletin 2025/25**

(21) Application number: **24217748.3**

(22) Date of filing: **05.12.2024**

(51) International Patent Classification (IPC):
**A61B 5/08** $^{(2006.01)}$     **A61B 5/00** $^{(2006.01)}$

(52) Cooperative Patent Classification (CPC):
**A61B 5/0816; A61B 5/6815; A61B 5/6898;**
**A61B 5/7257; A61B 5/7264;** A61B 5/0028

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(30) Priority: **12.12.2023  US 202318537436**

(71) Applicant: **STMicroelectronics International N.V.**
**1228 Plan-les-Ouates, Geneva (CH)**

(72) Inventors:
• **MAGNANI, Alessandro**
  **20152 MILANO (IT)**
• **RIZZARDINI, Federico**
  **20019 SETTIMO MILANESE (MI) (IT)**

(74) Representative: **Studio Torta S.p.A.**
**Via Viotti, 9**
**10121 Torino (IT)**

(54) **BREATH MONITORING ON TWS EARPHONES**

(57)    A wearable electronic device (102) detects the breathing of a user based on bone conduction of sounds waves. The wearable electronic device includes an inertial sensor unit (102). The inertial sensor unit generates sensor data based on bone conduction of sound. The inertial sensor unit generates frequency domain data based on the sensor data. The inertial sensor unit detects breathing of the user by performing a classification process based on the frequency domain data.

**FIG.2**

**Description**

TECHNICAL FIELD

The present disclosure relates to human breath detection based on bone conduction of sound detected by inertial MEMS sensors.

BACKGROUND

In many medical applications it is beneficial to reliably detect breathing of an individual. Typically, human breath detection (together with other biometric signals) is implemented in ad hoc devices that are usually positioned near the part of the body generating the signal (e.g., wearable bands, etc.). The devices are usually worn by the user only if ongoing measurement is needed.

In one possible solution, a human breath detection device detects breath with a microphone. However, microphones are electrically noisy, are subjected to external disturbances, and are often very expensive in terms of processing and energy resources. It can be difficult to efficiently detect human breathing with microphones.

BRIEF SUMMARY

An aim of the present invention is to provide a method, a sensor unit and a wearable electronic device for breath detection, that overcome the issues mentioned above.

According to the invention, a method, a sensor unit and a wearable electronic device are provided, as defined in the attached claims.

BRIEF DESCRIPTION OF THE DRAWINGS

For the understanding of the present invention, embodiments thereof are now described, purely as a non-limitative examples, with reference to the enclosed drawings, wherein:

Figure 1 is a block diagram of a system including a wearable electronic device that detects user breathing based on bone conduction of sound, in accordance with one embodiment.

Figure 2 is a functional block diagram of a control circuit of an inertial sensor unit of the wearable electronic device of Figure 1, in accordance with one embodiment.

Figures 3A-3C include graphs associated with frequency domain representation of inertial sensor data, in accordance with the embodiment of Figure 2.

Figure 4 is a flow diagram of a process for detecting human breathing, in accordance with another embodiment.

Figure 5 is an illustration of a user with a wearable electronic device, in accordance with one embodiment.

Figure 6 is an illustration of a user with a wearable electronic device, in accordance with another embodiment.

Figure 7 is a flow diagram of a method for detecting human breathing, in accordance with one embodiment.

Figure 8 is a flow diagram of a method for detecting human breathing, in accordance with another embodiment.

DETAILED DESCRIPTION

Unless the context requires otherwise, throughout the specification and claims which follow, the word "comprise" and variations thereof, such as, "comprises" and "comprising" are to be construed in an open, inclusive sense, that is as "including, but not limited to." Further, the terms "first," "second," and similar indicators of sequence are to be construed as interchangeable unless the context clearly dictates otherwise.

Reference throughout this specification to "one embodiment" or "an embodiment" means that a particular feature, structure or characteristic described in connection with the embodiment is included in at least one embodiment. Thus, the appearances of the phrases "in one embodiment" or "in an embodiment" in various places throughout this specification are not necessarily all referring to the same embodiment. Furthermore, the particular features, structures, or characteristics may be combined in any suitable manner in one or more embodiments.

As used in this specification and the appended claims, the singular forms "a," "an," and "the" include plural referents unless the content clearly dictates otherwise. It should also be noted that the term "or" is generally employed in its broadest sense, that is as meaning "and/or" unless the content clearly dictates otherwise.

Embodiments of the present disclosure utilize low power MEMS inertial sensors to detect human breathing via bone conduction of sound. A sensor unit including a MEMS sensor can be embedded in a common wearable electronic device such as earphones, headphones, smart glasses, or other types of electronic devices in contact with the body of the user. The sensor unit processes inertial sensor data from the inertial sensor and generates a frequency spectrum representation of the sensor data. The sensor unit extracts frequency spectrum features based on the bone conduction of sound and identifies periods of human breathing based on the frequency spectrum features.

In one embodiment, a method includes generating, with an inertial sensor unit of an electronic device worn by a user, sensor data based on bone conduction of sound, generating, with the inertial sensor unit, frequency domain data based on the sensor data, and detecting, with the inertial sensor unit, breathing of the user by performing a classification process based on the frequency domain data.

In one embodiment, the method includes calculating, from the frequency domain data, a spectral energy, a spectral centroid frequency, and a spectral spread based on the sensor data and detecting, with the inertial sensor unit, breathing of the user by performing a classification process based on the spectral energy, the spectral centroid frequency, and the spectral spread.

In one embodiment, a wearable electronic device includes a first sensor unit including an inertial sensor configured to generate first sensor data based on bone conduction of sound and a control circuit. The control circuit is configured to generate frequency domain data based on the first sensor data and generate breathing detection data indicative of breathing of the user based on the spectral energy, the spectral centroid frequency, and the spectral spread.

In one embodiment, a method includes generating, with an inertial sensor unit of an electronic device worn by a user, sensor data based on bone conduction of sound and performing an axis fusion process on the sensor data to fuse multiple axes in the sensor data. The method includes generating, from the sensor data, a plurality of windows, calculating, with the inertial sensor unit for each window, a first feature and a second feature, and detecting, with the inertial sensor unit, breathing of the user based on the first feature and the second feature of a plurality of windows.

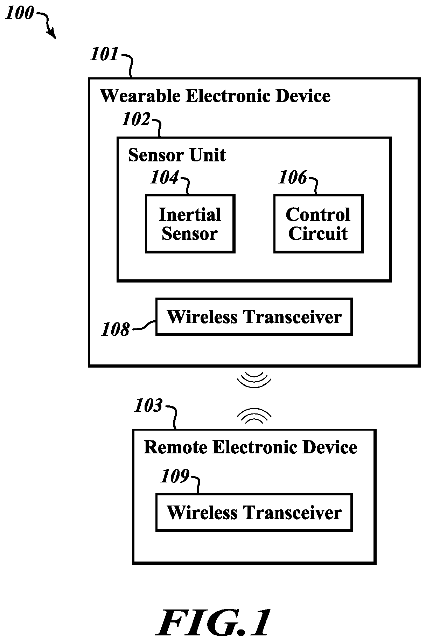

Figure 1 is a block diagram 100 of a system for detecting human breathing, in accordance with one embodiment.

The system 100 includes a wearable electronic device 101.

As will be set forth in more detail below, the components of the wearable electronic device 101 cooperate to detect breathing of a user that is wearing the wearable electronic device 101.

The wearable electronic device 101 is a device that can be coupled to or worn on the body of an individual. When used by an individual, the wearable electronic device 101 may be in continuous contact with the skin of the individual.

The wearable electronic device 101 can include wireless earphones, a headphone, smart glasses, or other types of wearable electronic devices.

When an individual speaks, the sounds made by the user are generated as soundwaves corresponding to vibrations produced in the vocal cords. The soundwaves may proceed to travel through the air. Other people may hear the words spoken by the individual when their ears sense the vibrations of the soundwaves carried through the air.

Soundwaves are also conducted by media other than air. Soundwaves are conducted by liquid and solid materials as well. Human bones conduct soundwaves in a peculiar manner. Accordingly, when a human speaks, the vibrations are conducted through the bones of the human.

Human breathing also generates soundwaves. One way to detect human breathing is to place a microphone at a location that can sense the soundwaves that travel through the air. However, human breathing is relatively quiet and detecting of breathing may call for highly sensitive microphones. Furthermore, background noises may interfere with the ability of a microphone to detect human breathing.

The wearable electronic device 101 detects the breathing of a user based on the bone conduction of soundwaves generated by the user's breathing. Because the electronic device 101 is in contact with the skin of the user, the electronic device 101 can reliably sense the bone conduction of sound resulting from breathing of the user.

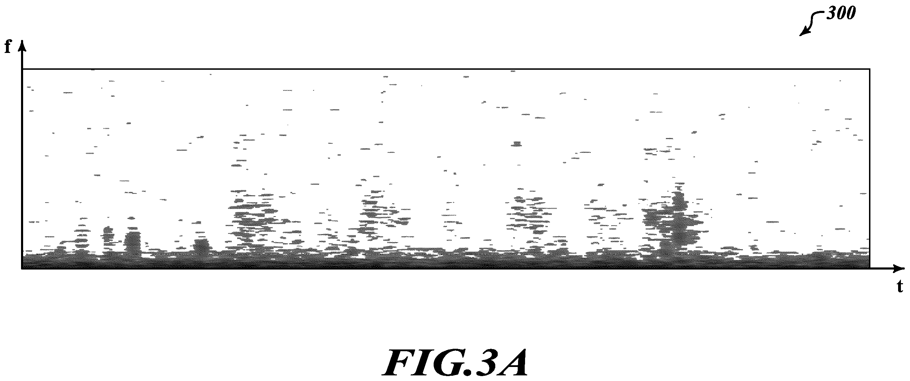

Soundwaves resulting from breathing may generally have a frequency range between 150 Hz and 450 Hz which visually appears (refer to Figures 3A-3C) as noise floor clouds. Frequencies below 150 Hz may correspond to external disturbances, such as human movement, and may be ignored when detecting breathing via bone conduction.

In particular, the wearable electronic device includes a sensor unit 102.

The sensor unit 102 includes an inertial sensor 104. The inertial sensor 104 senses vibrations resulting from the bone conduction of the sound of the user's breathing. The inertial sensor 104 can include a micro-electromechanical system (MEMS) sensor.

In one embodiment, the inertial sensor 104 includes an accelerometer. The accelerometer can be a three-axis accelerometer that senses acceleration in each of three mutually orthogonal sensing axes. The sensing axes may correspond to X, Y, and Z axis. The inertial sensor 104 may also include a gyroscope that senses rotation around three mutually orthogonal sensing axes.

The sensor unit 102 also includes a control circuit 106 coupled to the inertial sensor 104. The control circuit 106 can include processing resources, memory resources, and communication resources. As will be set forth in more detail below, the control circuit 106 is able to detect breathing of the user based on the bone conduction of sound sensed by the inertial sensor 104.

In one embodiment, the inertial sensor 104 is implemented in a first integrated circuit die. The control circuit 106 is implemented in a second integrated circuit die directly coupled to the first integrated circuit die.

In one embodiment, the inertial sensor 104 and the control circuit 106 are implemented in a single integrated circuit die as a system on-chip. The control circuit 106 may correspond to an application specific integrated circuit (ASIC).

The inertial sensor 104 initially generates analog sensor signals based on the bone conduction of soundwaves. The vibrations may be sensed capacitively, piezo-electrically, or in another suitable manner. The inertial sensor 104 generates the analog sensor signals based on the sensing.

The analog sensor signals are converted to digital sensor data by an analog-to-digital converter (ADC) either in the inertial sensor 104 or in the control circuit 106. The ADC can be configured to generate a selected number of samples of sensor data per second.

In one embodiment, the control circuit 106 receives the sensor data (or generates the sensor data from the sensor signals) and processes the sensor data in order to detect breathing of the user. The control circuit 106 can include signal processing circuitry to perform multiple processing steps to format or condition the sensor data in order to detect breathing of the user.

In one embodiment, the control circuit 106 generates frequency domain data from the sensor data. The sensor data is generally in a time domain (i.e., sequential samples). The control circuitry 106 generates the frequency domain data by converting the sensor data from the time domain to a frequency domain. Accordingly, as used herein, frequency domain data corresponds to sensor data that has been transformed to the frequency domain. The frequency domain data can include spectral data indicating the frequencies present in the groups of samples of the sensor data.

In one embodiment, the control circuitry 106 performs a discrete Fourier transform (DFT) on the sensor data in order to convert the sensor data to frequency domain data.

Alternatively, the control circuitry 106 can perform other types of transforms or conversions to generate frequency domain data. For example, the control circuitry can utilize other types of Fourier transforms, wavelet transforms, or other types of transforms to generate frequency domain data. In one embodiment, the control circuitry 106 performs a sliding discrete Fourier transform (SDFT).

The frequency domain data may be termed "spectral data".

In one embodiment, the control circuitry 106 generates a plurality of spectral features from the frequency domain data. The control circuitry 106 can then analyze the spectral features in order to detect the breathing of the user. Analyzing the spectral features can include performing a classification process based on the spectral features.

For example, the control circuitry 106 generates a spectral energy value $X_E$ for each group of samples, based on the spectral data. The spectral energy value corresponds to a spectral feature. In one embodiment, the control circuitry 106 generates the spectral energy value in the following manner:

$$X_E(n) = \sum_{k=bin\_start}^{bin\_stop} X_{k_r}^2 + X_{k_i}^2$$

where $X_{kr}$ is the real component of the Fourier component with index k, $X_{ki}$ is the imaginary component of the Fourier component with index k, bin_start corresponds to the beginning frequency bin and bin_stop corresponds to the final frequency bin. A spectral energy value $X_e$ (not present in the formula above, but present in the formulas below) is generated for each of n bins.

In the example of bone conduction of sound, a first frequency bin may be around 150 Hz and the final frequency bin may be around 450 Hz, though other frequency ranges can be utilized.

In one embodiment, the control circuitry 106 generates a spectral centroid frequency (SCF) value for each group of samples, based on the spectral data. The spectral centroid frequency value corresponds to a spectral feature. The spectral centroid frequency value can correspond to a center of mass of the spectrum for a group of samples.

$$SCF(n) = \sum_{k=bin\_start}^{bin\_size} \frac{k\,(X_{k_r}^2 + X_{k_i}^2)}{X_e}$$

For example, the control circuitry 106 generates the spectral centroid frequency value in the following manner: In one embodiment, the control circuitry 106 generates a spectral spread value (SSP) for each group of samples, based on the spectral data. The spectral spread value corresponds to a spectral feature. The spectral spread can correspond to the range of frequencies present within a particular group of samples.

For example, the spectral spread can be generated in the following manner:

$$SSP(n) = \sqrt{\sum_{k=bin\_start}^{bin\_size} \frac{(d_f k - SCF)^2 (X_{k_r}^2 + X_{k_i}^2)}{X_e}}$$

where dfk corresponds to the frequency corresponding to bin k.

In one embodiment, the control circuit 106 detects breathing by performing a classification process or detection process based on the features generated from the spectral data. For example, a breathing detection process can detect breathing for group of samples by comparing one or more of the features generated from the spectral data to one or more threshold values. The classification process may correspond to a breathing detection algorithm.

In one embodiment, the control circuit 106 includes an analysis model trained with a machine learning process to detect human breathing. The analysis model can be trained using a supervised machine learning process to detect human breathing based on one or more of the raw sensor data, the spectral data, the spectral features, or time domain features. Accordingly, the analysis model can be trained based on a training set gathered from the user of the wearable electronic device 100, based on a training set gathered from users of other wearable electronic devices 100, or based on other types of training sets. The analysis model can output a classification indicating whether or not the group of samples is indicative of human breathing.

In one embodiment, human breathing is detected based on multiple groups of samples of sensor data. Each group may correspond to a frame or a window of samples of sensor data. The breathing detection processes may output a pre-classification for each window or frame of sensor data. An overall classification of breathing may be generated for a plurality of windows or frames of sensor data.

For example, breathing detection may correspond to detecting whether the wearer is inhaling. Inhalation may occur over a large number of frames or windows of sensor data. The breathing detection process may generate a classification of inhalation for a plurality of consecutive windows or frames. This may correspond to detecting the range of time during which the user was inhaling. The pre-classification from each window or frame may be utilized in determining whether a group of consecutive frames represents inhalation.

A similar type of classification can be made for exhalation rather than inhalation. Various other types of classification processes can be utilized.

In one embodiment, the windows or frames are furtherly processed through a windowing function.

The windowing function may correspond to the Hann windowing function. The Hann windowing function start and end at a value of zero with a value of one in the center. Other types of window functions can be applied to furtherly process the windows or frames.

The wearable electronic device 101 can also include a wireless transceiver 108. The wireless transceiver 108 can receive breathing detection data from the sensor unit 102 and can transmit the breathing detection data to a remote electronic device 103.

The wireless transceiver 108 can operate in accordance with one or more wireless protocols including Bluetooth, Wi-Fi, or other suitable wireless communication protocols.

The remote electronic device 103 can receive breathing detection data from the wearable electronic device 101.

The remote electronic device 103 can display reading data to the user.

The remote electronic device 103 can include one or more applications or circuits that process the breathing data in order to perform one or more health monitoring related functions.

The remote electronic device 103 can include a smartphone, a smartwatch, a laptop computer, a tablet, or other types of electronic devices.

The remote electronic device 103 can include a wireless transceiver 109 that can receive the breathing detection data from the wearable electronic device 101. The wireless transceiver 109 can also transmit data to the wearable electronic device 101.

The wireless transceiver 109 can operate in accordance with one or more wireless protocols including Bluetooth, Wi-Fi, or other suitable wireless communication protocols.

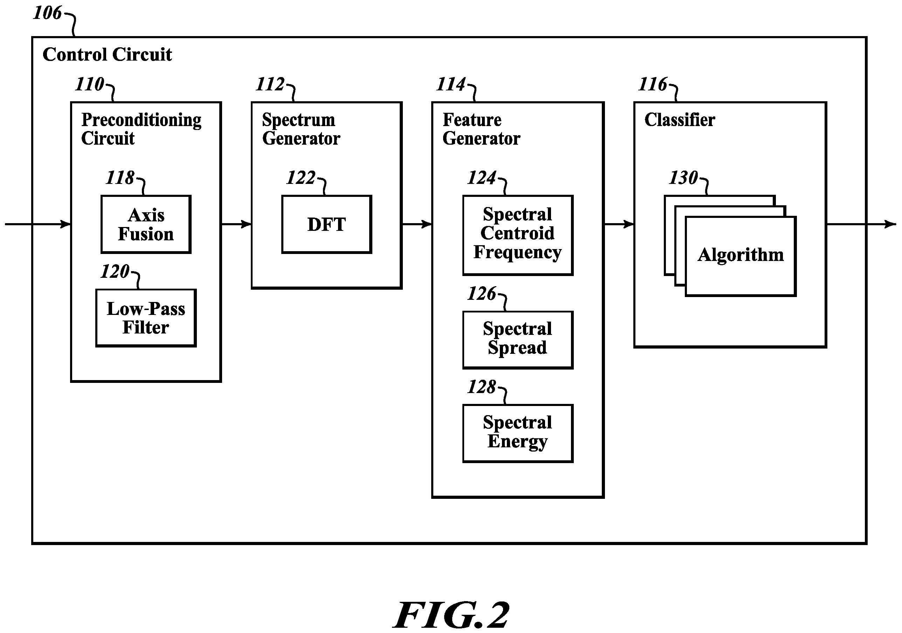

Figure 2 is a block diagram of the control circuit 106, according to one embodiment. The control circuit 106 of Figure 2 is one example of a control circuit 106 of Figure 1.

The control circuit 106 includes a preconditioning circuit 110, a spectrum generator 112, a feature generator 114, and a classifier 116.

The preconditioning circuit 110 receives the sensor data from the inertial sensor 104.

The preconditioning circuit 110 includes an axis fusion module 118. The axis fusion module 118 performs an axis fusion process on the sensor data.

In an embodiment in which the control circuit 106 detects human breathing based on spectral features, the data associated with any particular axis of the inertial sensor 104 may be less beneficial than the total magnitude of the signals for all of the axes combined. Accordingly, the axis fusion module 118 may generate an absolute value of the magnitude of each sample of the sensor data by taking the square root of the sum of the square of the sensor data on each axis. This retains frequency information while simplifying the data set. The axis fusion module may perform a classic norm or magnitude computation to mix the vibration contributions from all the axes. Alternatively, the axis fusion module 118 can be configured to just select the value generated by a specific axis and ignore the value generated by the other axes.

In one embodiment, the preconditioning circuit 110 includes a low-pass filter module 120. The low-pass filter module 120 performs a low-pass filtering process on the fused sensor data (i.e., the sensor data as modified by the axis fusion module 118). The low-pass filter process can correspond to a CIC filter that also decimates the sensor data. The low-pass filter may have a 1 kHz bandwidth, in one example.

The preconditioning circuit 110 can also include other circuitry and perform other functions.

In one embodiment, the spectrum generator 112 includes a discrete Fourier transform module 122. The discrete Fourier transform (DFT) module 122 receives the filtered sensor data from the low-pass filter 120 of the preconditioning circuit 110. The DFT module 122 generates spectral data from the sensor data by performing a discrete Fourier transform on the sensor data. The spectral data can be output based on the number of samples in each window.

Other types of processes can be utilized to generate spectral data from the sensor data. The feature generator 114 receives the spectral data from the DFT module 122.

In one embodiment, the feature generator 114 includes a spectral centroid frequency module 124. The spectral centroid frequency module 124 generates the spectral centroid frequency, as described previously in relation to Figure 1.

In one embodiment, the feature generator 114 includes a spectral spread module 126. The spectral spread module 126 generates the spectral spread value as described previously in relation to Figure 1.

In one embodiment, the feature generator 114 includes a spectral energy module 128. The spectral energy module 128 generates spectral energy values as described previously in relation to Figure 1.

The feature generator 114 can include other circuitry or generate other features than those described herein.

In one embodiment, the classifier 116 includes one or more classification process modules 130. The one or more classification process modules can perform one or more classification processes or breathing detection processes based on the feature data generated by the feature generator 114.

In one embodiment, a classification process includes generating a value as a function of the spectral energy $X_E$, the spectral centroid frequency SCF and the spectral spread SSP and comparing the value to a threshold.

For example, breathing is detected by generating an output value (out) and comparing the output value to a threshold value (th) in the following manner:

$$out = \frac{X_E(n)}{SCF(n) \cdot SSP(n)} > th$$

In this example, if the output value is greater than the threshold, then breathing is detected. This metric is proportional to the noise floor cloud presence. The threshold can be used to discriminate the real breathing activity.

Other types of mathematical formulas may be utilized.

In one embodiment, a classification process includes utilizing a plurality of threshold values. In particular, the classification process can compare the spectral energy to a first threshold (th1), the spectral centroid frequency to a second threshold (th2), and the spectral spread to a third threshold th3 in the following manner:

$$out = (X_E(n) > th1) \ and \ (SCF(n) \leq th2) \ and \ (SSP(n) \leq th3)$$

In this case, breathing is detected if the spectral energy is greater than the first threshold, the spectral centroid frequency is less than or equal to the second threshold, and the spectral spread is less than or equal to the third threshold. This process may correspond to a binary tree process. The results are Boolean values combined to satisfy the breath activity detection.

In one embodiment, a classification process includes an analysis model, such as a neural network, trained with a machine learning process to detect and classify breathing based on spectral features.

In one embodiment, the analysis model receives the spectral energy, the spectral centroid frequency, and the spectral spread and detects breathing based on these features.

Other types of features and other types of analysis models can be utilized for detecting breathing.

A neural network inference can be trained to mix the three spectral features to detect breathing activity. Fully connected, convolutional or recurrent neural network layers can be utilized to take into account timing envelopes. The neural network can include a dense layer that can assemble the previous layer outputs and generate single breath detection or prediction.

Figure 3A is a graph 300 illustrating spectral data versus time, in accordance with one embodiment. The x-axis corresponds to time and the y-axis corresponds to frequency.

In one example, the frequency is between 100 Hz and 800 Hz, though other frequencies ranges can be utilized.

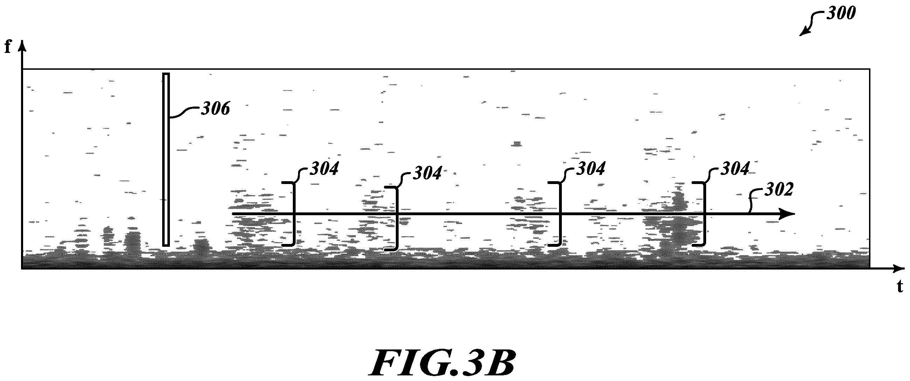

Figure 3B is the graph 300 of Figure 3A, but with markers highlighting spectral features, in accordance with one embodiment. The marker 306 corresponds to the spectral energy $X_E$. The markers 304 correspond to the spectral spread SSP, and the marker 302 corresponds to the spectral centroid frequency SCF.

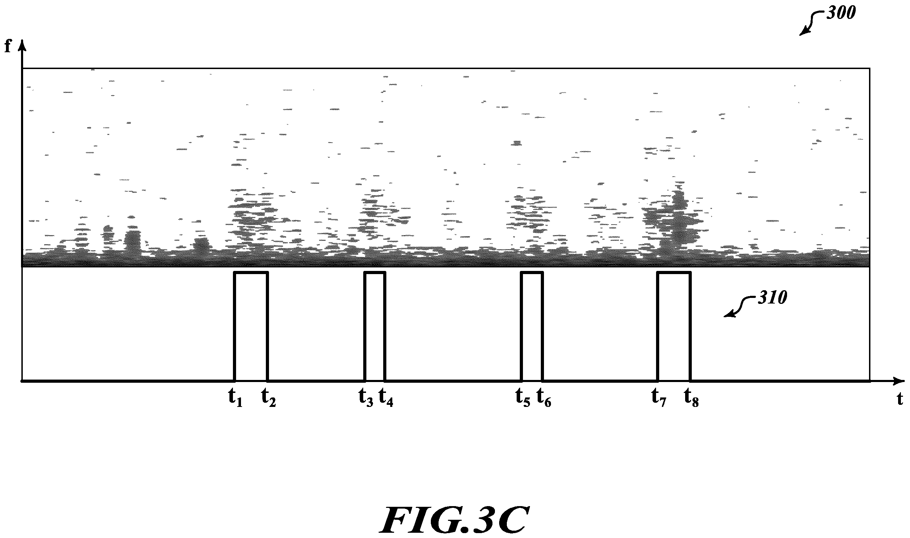

Figure 3C corresponds to the graph 300 including a breathing indication graph 310 positioned below the graph 300, in accordance with one embodiment. The graph 310 includes pulses indicating periods of time during which breathing is detected. The pulses may correspond to inhale stages during which a user of the wearable electronic device 100 is inhaling.

Four periods of inhalation are shown in Figure 3C. The user is inhaling between times $t_1$ and $t_2$, between times $t_3$ and $t_4$, between times $t_5$ and $t_6$, and between times $t_7$ and $t_8$. Accordingly, there are four periods of inhalation in Figure 3C.

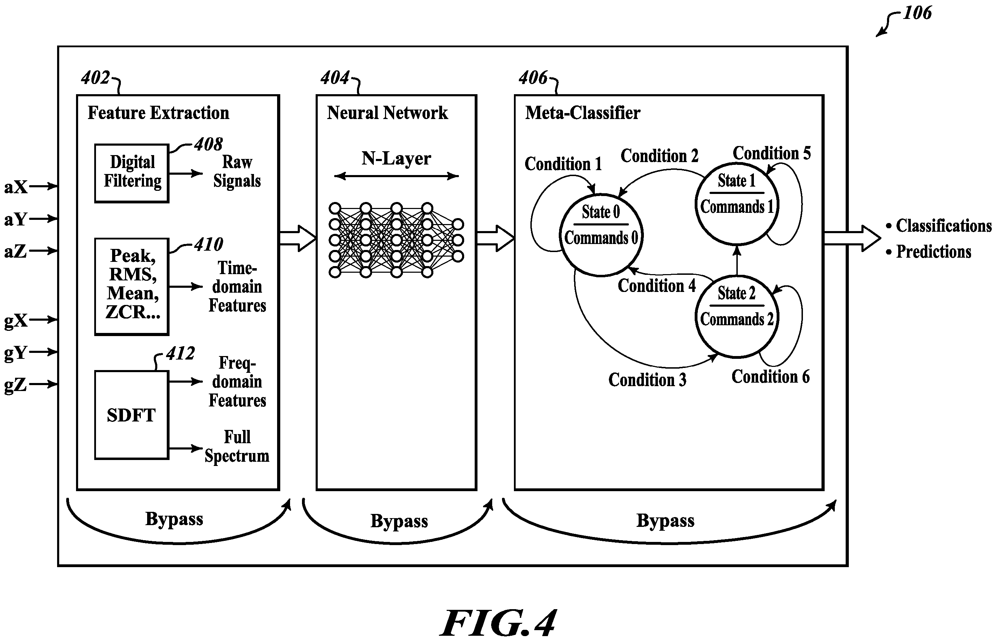

Figure 4 is a block diagram of a control circuit 106 in accordance with another embodiment. The control circuit 106 is one example of a control circuit 106 of Figure 1.

The control circuit 106 receives raw sensor signals, in particular including measurements along three axes of accelerometer signals aX, aY, and aZ (which can be indicative of bone conduction of sound) and measurements along three axes of gyroscope signals gX, gY, and gZ. In some cases, only the acceleration data may be utilized.

In detail, the control circuit 106 comprises a feature extraction module 402 that performs feature extraction of the sensor signals. The feature extraction can include digital filtering of the sensor data with the digital filter 408.

The feature extraction includes generation of time domain features from the sensor data with a time domain feature module 410. Time domain features can include peak-to-peak, root mean square (RMS), mean, variance, energy, maximum, minimum, zero-crossing rate (ZCR), or other types of time domain features.

The feature extraction includes generation of frequency domain signal and frequency domain features using the sliding discrete Fourier transform (SDFT) module 412. This can include performing a Fourier transform and generating spectral energy, spectral centroid frequency, and spectral spread data as described previously.

The control circuit 106 also includes a neural network 404.

The neural network 404 can receive one or more of the raw sensor data, the time domain features, the frequency domain features, and the full spectral data from the feature extraction module 402. The neural network then analyzes the various data and features in order to generate a pre-classification. The pre-classification can indicate, for each frame or window, whether or not breathing is detected. Other types of analysis models other than neural networks can be utilized.

As shown in Figure 4, in one embodiment, the feature extraction is bypassed and the sensor data is provided directly to the neural network.

The control circuit 106 also includes a meta-classifier 406 that receives the pre-classification data from the neural network 404.

The meta-classifier can include a plurality of conditions, states, and commands. The meta-classifier can generate classifications (i.e., breathing detected) based the pre-classification provided by the neural network, as well as based on the data provided by the feature extraction.

As shown in Figure 4, the neural network 404 can be bypassed and the feature data from the feature extraction can be provided directly to the meta-classifier. Furthermore, the meta-classifier 406 can also be bypassed such that the neural network provides classification.

In the example of Figure 4, the meta-classifier 406 includes three states, three commands, and six conditions, on the basis of which classifications and predictions can be made. However, other numbers of states, commands, and conditions can be utilized. Various other types of processes and configurations can be utilized.

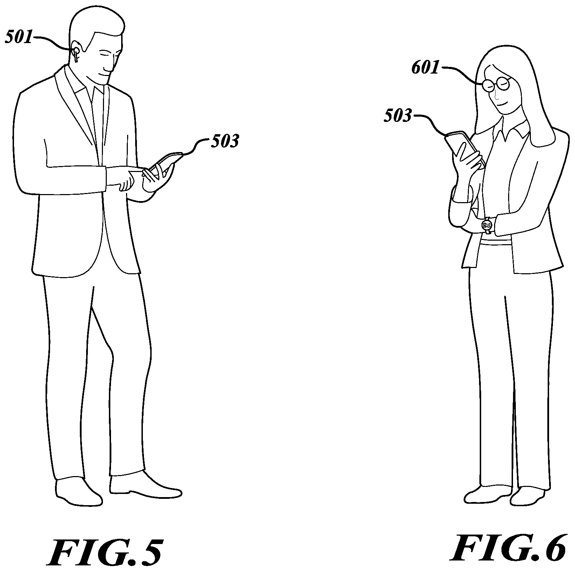

Figure 5 is an illustration of an individual wearing earphones 501 and holding a smartphone 503, in accordance with one example. The earphones 501 are one example of a wearable electronic device 101 of Figure 1. The earphones 501 are in contact with the skin of the user. The smartphone 503 is one example of a remote electronic device 103 of Figure 1. Although only a single earphone 501 is apparent in Figure 5, in practice, the individual may wear two earphones 501.

In one embodiment, the sensor unit 102 including the inertial sensor 104 and the control circuit 106 are included in a single one of the earphones 501. That earphone 501 detects breathing of the user based on bone conduction of sound as described previously.

In one embodiment, the second earphone 501 may also generate sensor data and provide the sensor data to the first earphone 501. The first earphone 501 may then detects breathing based on the sensor data from both the first earphone 501 and the second earphone 501.

The earphones 501 can provide breathing detection data to the smartphone 503. The smartphone 503 may utilize the breathing detection data in one or more applications or circuits of the smartphone 503.

The smartphone 503 may display breathing detection data to the user. The smartphone 503 may generate health or wellness data based on the breathing detection data. The smartphone 503 may generate one or more reports, graphs, images, or other data that can be displayed or provided to the user or that can be provided to other systems.

Figure 6 is an illustration of an individual wearing smart glasses 601 and holding a smartphone 503, in accordance with another example. The smart glasses 601 is one example of a wearable electronic device 101 of Figure 1. The smart glasses 601 is in contact with the skin of the user. The smartphone 601 is one example of a remote electronic device 103 of Figure 1.

In one embodiment, the sensor unit 102 including the inertial sensor 104 and the control circuit 106 are included in the smart glasses 601. The smart glasses 601 detects breathing of the user based on bone conduction of sound as described previously.

In one embodiment, the smart glasses 601 can display breathing detection data to the user. The smart glasses 601 may utilize the breathing detection data in one or more applications or circuits.

The smart glasses 601 can provide breathing detection data to the smartphone 503. The smartphone 503 may utilize the breathing detection data in one or more applications or circuits of the smartphone 503.

The smartphone 503 may display breathing detection data to the user. The smartphone 503 may generate health or wellness data based on the breathing detection data. The smartphone 503 may generate one or more reports, graphs, images, or other data that can be displayed are provided to the user or that can be provided to other systems.

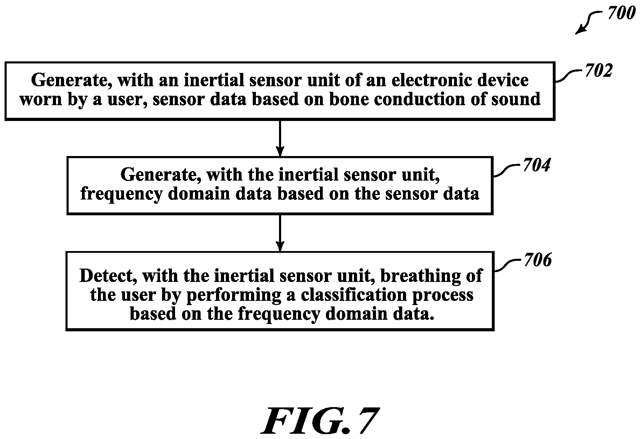

Figure 7 is a flow diagram of a method for detecting breathing with a wearable electronic device.

The method 700 can utilize components, processes, and systems described in relation to Figures 1-6.

At step 702, the method 700 includes generating, with an inertial sensor unit of an electronic device worn by a user, sensor data based on bone conduction of sound.

At step 704, the method includes generating, with the inertial sensor unit, frequency domain data based on the sensor data.

At step 706, the method includes detecting, with the inertial sensor unit, breathing of the user by performing a classification process based on the frequency domain data.

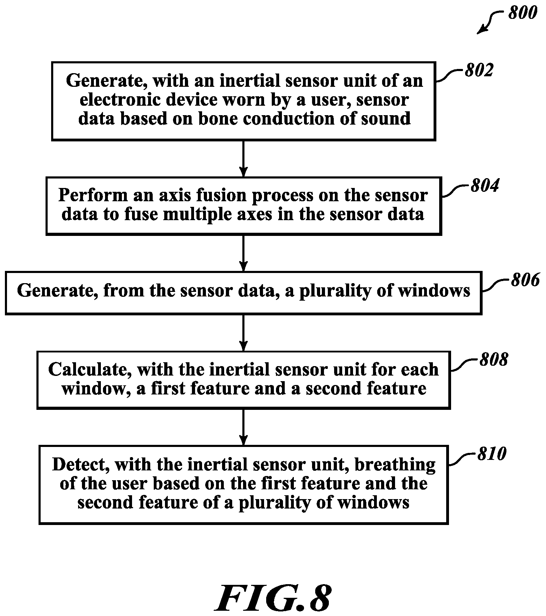

Figure 8 is a flow diagram of a method for detecting breathing with a wearable electronic device.

The method 800 can utilize components, processes, and systems described in relation to Figures 1-6.

The method 800 can also be a particular implementation of the method 700 of Figure 7, wherein the steps 704 and 706 are executed for several windows and for a plurality of features, as better defined in the following.

At step 802, the method 800 includes generating, with an inertial sensor unit of an electronic device worn by a user, sensor data based on bone conduction of sound.

At step 804, the method 800 includes performing an axis fusion process on the sensor data to fuse multiple axes in the sensor data.

At step 806, the method 800 includes generating, from the sensor data, a plurality of windows.

At step 808, the method 800 includes calculating, with the inertial sensor unit for each window, a first feature and a second feature.

At step 810, the method 800 includes detecting, with the inertial sensor unit, breathing of the user based on the first feature and the second feature of a plurality of windows.

In one embodiment, a method includes generating, with an inertial sensor unit of an electronic device worn by a user, sensor data based on bone conduction of sound, generating, with the inertial sensor unit, frequency domain data based on the sensor data, and detecting, with the inertial sensor unit, breathing of the user by performing a classification process based on the frequency domain data.

In one embodiment, the method includes calculating, from the frequency domain data, a spectral energy, a spectral centroid frequency, and a spectral spread based on the sensor data and detecting, with the inertial sensor unit, breathing of the user by performing a classification process based on the spectral energy, the spectral centroid frequency, and the spectral spread.

In one embodiment, the method includes performing axis fusion on the sensor data with a norm computation prior to generating the frequency domain data.

In one embodiment, the method includes performing low-pass filtering and decimation after performing axis fusion and prior to generating the frequency domain data.

In one embodiment, the method includes generating, from the sensor data, a plurality of windows and generating the frequency domain data by performing a sliding discrete Fourier transform on each window.

In one embodiment, calculating the spectral energy, the spectral centroid frequency, and the spectral spread includes calculating the spectral energy, the spectral centroid frequency, and the spectral spread for each window.

In one embodiment, the classification process includes making a classification for each window from a group of the windows and detecting breathing for the group of windows based on the classification of each window of the group.

In one embodiment, the classification process includes generating a value by dividing the spectral energy by the product of the spectral centroid frequency and spectral spread and comparing the value to a threshold.

In one embodiment, the classification algorithm includes comparing the spectral energy to a first threshold value, comparing the spectral centroid frequency to a second threshold value, and comparing the spectral spread to a third threshold value.

In one embodiment, the method includes outputting, from the inertial sensor unit, breathing detection data based on detecting the breathing.

In one embodiment, the classification process includes passing the spectral energy, the spectral centroid frequency, and the spectral spread to an analysis model trained with a machine learning process and detecting breathing based on a classification of the analysis model.

In one embodiment, the method includes outputting breathing detection data from the wearable electronic device to a remote electronic device.

In one embodiment, a wearable electronic device includes a first sensor unit including an inertial sensor configured to generate first sensor data based on bone conduction of sound and a control circuit. The control circuit is configured to generate frequency domain data based on the first sensor data and generate breathing detection data indicative of breathing of the user based on the spectral energy, the spectral centroid frequency, and the spectral spread.

In one embodiment, the control circuit is configured to generate, from the frequency domain data, a spectral energy, a spectral centroid frequency, and a spectral spread based on the first sensor data and generate the breathing detection data based on the spectral energy, the spectral centroid frequency, and the spectral spread.

In one embodiment, the control circuit includes an analysis model trained with a machine learning process to detect breathing based on the spectral energy, the spectral centroid frequency, and the spectral spread.

In one embodiment, the electronic device includes a first earphone including the sensor unit.

In one embodiment, the electronic device includes a second earphone including a second sensor unit configured to provide second sensor data to the first sensor unit. The control circuit is configured to generate the breathing detection data based on the first sensor data and the second sensor data.

In one embodiment, a method includes generating, with an inertial sensor unit of an electronic device worn by a user, sensor data based on bone conduction of sound and performing an axis fusion process on the sensor data to fuse multiple axes in the sensor data. The method includes generating, from the sensor data, a plurality of windows, calculating, with the inertial sensor unit for each window, a first feature and a second feature, and detecting, with the inertial sensor unit, breathing of the user based on the first feature and the second feature of a plurality of windows.

In one embodiment, the method includes calculating, with the inertial sensor unit for each window, a third feature and detecting, with the inertial sensor unit, breathing of the user based on the first feature, the second feature, and the third feature of a plurality of windows.

In one embodiment, the first feature is spectral energy, the second feature is a spectral centroid frequency, and the third feature is spectral spread.

From what has been described and illustrated previously, the advantages of the present invention are evident.

Finally, it is clear that modifications and variations may be made to what has been described and illustrated herein, without thereby departing from the scope of the present invention, as defined in the annexed claims. For example, the various embodiments described above can be combined to provide further embodiments. These and other changes can be made to the embodiments in light of the above-detailed description.

Examples of the present disclosure may be summarized as follows.

1. A method, comprising:

generating, with an inertial sensor unit of an electronic device worn by a user, sensor data based on bone conduction of sound;
generating, with the inertial sensor unit, frequency domain data based on the sensor data; and
detecting, with the inertial sensor unit, breathing of the user by performing a classification process based on the frequency domain data.

2. The method of example 1, comprising:

calculating, from the frequency domain data, a spectral energy, a spectral centroid frequency, and a spectral spread based on the sensor data; and
detecting, with the inertial sensor unit, breathing of the user by performing a classification process based on the spectral energy, the spectral centroid frequency, and the spectral spread.

3. The method of example 2, comprising performing axis fusion on the sensor data prior to generating the frequency domain data.

4. The method of example 3, comprising performing low-pass filtering and decimation after performing axis fusion and prior to generating the frequency domain data.

5. The method of example 2, comprising:

generating, from the sensor data, a plurality of windows; and
generating the frequency domain data by performing a sliding discrete Fourier transform on each window.

6. The method of example 5, wherein calculating the spectral energy, the spectral centroid frequency, and the spectral spread includes calculating the spectral energy, the spectral centroid frequency, and the spectral spread for each window.

7. The method of example 6, wherein the classification process includes:

    making a classification for each window from a group of the windows; and
    detecting breathing for the group of windows based on the classification of each window of the group.

8. The method of example 2, wherein the classification process includes:

    generating a value by dividing the spectral energy by the product of the spectral centroid frequency and spectral spread; and
    comparing the value to a threshold.

9. The method of example 2, wherein the classification algorithm includes:

    comparing the spectral energy to a first threshold value;
    comparing the spectral centroid frequency to a second threshold value; and
    comparing the spectral spread to a third threshold value.

10. The method of example 2, comprising outputting, from the inertial sensor unit, breathing detection data based on detecting the breathing.

11. The method of example 2, wherein the classification algorithm includes:

    passing the spectral energy, the spectral centroid frequency, and the spectral spread to an analysis model trained with a machine learning process; and
    detecting breathing based on a classification of the analysis model.

12. The method of example 11, comprising outputting breathing detection data from the wearable electronic device to a remote electronic device.

13. A wearable electronic device, comprising:
a first sensor unit including:

    an inertial sensor configured to generate first sensor data based on bone conduction of sound;
    a control circuit configured to:

        generate frequency domain data based on the first sensor data;
        generate breathing detection data indicative of breathing of the user based on the spectral energy, the spectral centroid frequency, and the spectral spread.

14. The wearable electronic device of example 13, wherein the control circuit is configured to generate, from the frequency domain data, a spectral energy, a spectral centroid frequency, and a spectral spread based on the first sensor data and generate the breathing detection data based on the spectral energy, the spectral centroid frequency, and the spectral spread.

15. The electronic device of example 13, wherein the control circuit includes an analysis model trained with a machine learning process to detect breathing based on the spectral energy, the spectral centroid frequency, and the spectral spread.

16. The electronic device of example 15, comprising a first earphone including the sensor unit.

17. The electronic device of example 16, comprising a second earphone including a second sensor unit configured to provide second sensor data to the first sensor unit, wherein the control circuit is configured to generate the breathing detection data based on the first sensor data and the second sensor data.

18. A method, comprising:

    generating, with an inertial sensor unit of an electronic device worn by a user, sensor data based on bone conduction of sound;
    performing an axis fusion process on the sensor data to fuse multiple axes in the sensor data;
    generating, from the sensor data, a plurality of windows;
    calculating, with the inertial sensor unit for each window, a first feature and a second feature; and

detecting, with the inertial sensor unit, breathing of the user based on the first feature and the second feature of a plurality of windows.

19. The method of example 18, comprising:

calculating, with the inertial sensor unit for each window, a third feature; and
detecting, with the inertial sensor unit, breathing of the user based on the first feature, the second feature, and the third feature of a plurality of windows.

20. The method of example 19, wherein the first feature is spectral energy, the second feature is spectral centroid frequency, and the third feature is spectral spread.


**Claims**

1. A method (700, 800), comprising:

generating (702, 802), with an inertial sensor unit (102) of an electronic device (101) worn by a user, sensor data based on bone conduction of sound;
generating (704), with the inertial sensor unit, frequency domain data based on the sensor data; and
detecting (706), with the inertial sensor unit, breathing of the user by performing a classification process based on the frequency domain data.

2. The method of claim 1, wherein the step of generating the frequency domain data comprises calculating, from the sensor data, a spectral energy (128), a spectral centroid frequency (124), and a spectral spread (126) based on the sensor data, and
wherein the step of detecting the breathing of the user comprises detecting, with the inertial sensor unit, breathing of the user by performing a classification process based on the spectral energy, the spectral centroid frequency, and the spectral spread.

3. The method of claim 1 or 2, comprising outputting, from the inertial sensor unit, breathing detection data based on detecting the breathing, in particular to a remote electronic device (103).

4. The method of anyone of the previous claims, wherein the classification process includes at least one of the following:

generating a value by dividing the spectral energy by the product of the spectral centroid frequency and spectral spread, and comparing the value to a threshold;
comparing the spectral energy to a first threshold value, comparing the spectral centroid frequency to a second threshold value, and comparing the spectral spread to a third threshold value;
passing the spectral energy, the spectral centroid frequency, and the spectral spread to an analysis model trained with a machine learning process, and detecting breathing based on a classification of the analysis model.

5. The method of anyone of the previous claims, comprising:

generating, from the sensor data, a plurality of windows; and
generating the frequency domain data by performing a sliding discrete Fourier transform on each window, wherein calculating the spectral energy, the spectral centroid frequency, and the spectral spread includes calculating the spectral energy, the spectral centroid frequency, and the spectral spread for each window, wherein the classification process includes:

making a classification for each window from a group of the windows; and
detecting breathing for the group of windows based on the classification of each window of the group.

6. The method of anyone of the previous claims, comprising performing (804) axis fusion on the sensor data prior to generating the frequency domain data.

7. The method of claim 6, further comprising performing low-pass filtering and decimation after performing axis fusion and prior to generating the frequency domain data.

8. The method of claim 6 or 7, further comprising generating (806), from the sensor data, a plurality of windows,

   wherein the step of generating the frequency domain data comprises calculating (808), with the inertial sensor unit for each window, a first feature and a second feature of said frequency domain data, and
   wherein the step of detecting the breathing of the user comprises detecting (810), with the inertial sensor unit, breathing of the user based on the first feature and the second feature of a plurality of windows.

9. The method of claim 8, comprising:

   calculating, with the inertial sensor unit for each window, also a third feature; and
   detecting, with the inertial sensor unit, breathing of the user also based on the third feature of a plurality of windows.

10. The method of claim 9, wherein the first feature is spectral energy (128), the second feature is spectral centroid frequency (124), and the third feature is spectral spread (126).

11. A sensor unit (102) including:

    an inertial sensor (104) configured to generate first sensor data based on bone conduction of sound;
    a control circuit (106) configured to:

       generate frequency domain data based on the first sensor data;
       detect breathing of the user by performing a classification process based on the frequency domain data.

12. The sensor unit of claim 11, wherein, in order to detect breathing of the user, the control circuit is configured to:

    generate, from the frequency domain data, a spectral energy (128), a spectral centroid frequency (124), and a spectral spread (126) based on the first sensor data; and
    generate breathing detection data indicative of breathing of the user based on the spectral energy, the spectral centroid frequency, and the spectral spread.

13. The sensor unit of claim 12, wherein the control circuit includes an analysis model trained with a machine learning process to detect breathing based on the spectral energy, the spectral centroid frequency, and the spectral spread.

14. A wearable electronic device (101) comprising a first sensor unit (102) according to anyone of claims 11-13.

15. The wearable electronic device of claim 14, comprising a first earphone, including the first sensor unit, and a second earphone, including a second sensor unit (102), the second sensor unit being configured to provide second sensor data to the first sensor unit, wherein the control circuit is configured to generate the breathing detection data based on the first sensor data and the second sensor data.

*FIG.1*

100

101 Wearable Electronic Device

102 Sensor Unit

104 Inertial Sensor

106 Control Circuit

108 Wireless Transceiver

103 Remote Electronic Device

109 Wireless Transceiver

FIG.2

EP 4 570 174 A1

**FIG.3A**

EP 4 570 174 A1

**FIG.3B**

FIG.3C

EP 4 570 174 A1

**FIG.4**

FIG.6

FIG.5

⌐ 700

Generate, with an inertial sensor unit of an electronic device worn by a user, sensor data based on bone conduction of sound ⌐702

Generate, with the inertial sensor unit, frequency domain data based on the sensor data ⌐704

Detect, with the inertial sensor unit, breathing of the user by performing a classification process based on the frequency domain data. ⌐706

*FIG. 7*

800

```
┌─────────────────────────────────────┐
│  Generate, with an inertial sensor   │──802
│  unit of an electronic device worn   │
│  by a user, sensor data based on     │
│  bone conduction of sound            │
└─────────────────────────────────────┘
                  │
                  ▼
┌─────────────────────────────────────┐
│  Perform an axis fusion process on   │──804
│  the sensor data to fuse multiple    │
│  axes in the sensor data             │
└─────────────────────────────────────┘
                  │
                  ▼
┌─────────────────────────────────────┐
│  Generate, from the sensor data, a   │──806
│  plurality of windows                │
└─────────────────────────────────────┘
                  │
                  ▼
┌─────────────────────────────────────┐
│  Calculate, with the inertial sensor │──808
│  unit for each window, a first       │
│  feature and a second feature        │
└─────────────────────────────────────┘
                  │
                  ▼
┌─────────────────────────────────────┐
│  Detect, with the inertial sensor    │──810
│  unit, breathing of the user based   │
│  on the first feature and the        │
│  second feature of a plurality of    │
│  windows                             │
└─────────────────────────────────────┘
```

*FIG.8*

**EUROPEAN SEARCH REPORT**

**Application Number**

EP 24 21 7748

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2017/273621 A1 (CAMACHO PEREZ JOSE RODRIGO [MX] ET AL) 28 September 2017 (2017-09-28) * paragraphs [0034], [0038], [0049] - [0051]; figures 1-3, 8-11 * | 1-15 | INV. A61B5/08 A61B5/00 |
| X | WO 2022/261208 A2 (DATHOMIR LABORATORIES LLC [US]) 15 December 2022 (2022-12-15) * paragraphs [0040], [0096] - [0097], [0111] - [0113] * | 1-15 | |
| X | HAN FEIYU ET AL: "BreathSign: Transparent and Continuous In-ear Authentication Using Bone-conducted Breathing Biometrics", IEEE INFOCOM 2023 - IEEE CONFERENCE ON COMPUTER COMMUNICATIONS, IEEE, 17 May 2023 (2023-05-17), pages 1-10, XP034412277, DOI: 10.1109/INFOCOM53939.2023.10229037 [retrieved on 2023-08-30] * abstract; figures 1-2, 4 * * the whole document * | 1-15 | |

TECHNICAL FIELDS SEARCHED (IPC)

A61B

The present search report has been drawn up for all claims

| Place of search | Date of completion of the search | Examiner |
|---|---|---|
| The Hague | 23 April 2025 | Furlan, Stéphane |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 21 7748

This annex lists the patent family members relating to the patent documents cited in the above-mentioned European search report.
The members are as contained in the European Patent Office EDP file on
The European Patent Office is in no way liable for these particulars which are merely given for the purpose of information.

23-04-2025

| Patent document cited in search report | Publication date | Patent family member(s) | | Publication date |
|---|---|---|---|---|
| US 2017273621 A1 | 28-09-2017 | US | 2017273621 A1 | 28-09-2017 |
| | | WO | 2017165028 A1 | 28-09-2017 |
| WO 2022261208 A2 | 15-12-2022 | CN | 117479880 A | 30-01-2024 |
| | | US | 2024277260 A1 | 22-08-2024 |
| | | WO | 2022261208 A2 | 15-12-2022 |

EPO FORM P0459

For more details about this annex : see Official Journal of the European Patent Office, No. 12/82